Europäisches Patentamt

European Patent Office

Office européen des brevets

(19)

(11) Publication number: **0 228 787**

**A1**

## (12) EUROPEAN PATENT APPLICATION

(21) Application number: 86308643.5

(51) Int. Cl.⁴: **A61M 25/00 , A61M 1/10**

(22) Date of filing: 06.11.86

(30) Priority: 08.11.85 US 796273

(43) Date of publication of application:
15.07.87 Bulletin 87/29

(84) Designated Contracting States:
BE CH DE FR GB LI LU NL

(71) Applicant: DATASCOPE CORP.
580 Winters Avenue
Paramus New Jersey 07652(US)

(72) Inventor: Kontos, Stavros B.
11 Bear Brook Drive
Woodcliff Lake New Jersey 07675(US)

(74) Representative: Lerwill, John et al
A.A. Thornton & Co. Northumberland House
303-306 High Holborn
London, WC1V 7LE(GB)

(54) Prefolded balloon catheter.

(57) A prewrapped percutaneously insertable intra-aortic balloon catheter (10) and a kit including such a catheter, in which the balloon (18) is wrapped ultra-compactly, preferably by being flattened, folded over on itself along a longitudinal folding-line, and then rolled up around an elongated member (24) which extends the length of the balloon chamber. One or more sleeve-like retainers (70) may be fitted over the wrapped balloon. Thus, the physician need not have any part in the folding or wrapping of the balloon, or in the removal of a wrapping device from the balloon, since the latter can be unpacked and inserted into the patient without such steps.

Fig. 5A.

Fig. 5.

## PREFOLDED BALLOON CATHETER

### BACKGROUND OF THE INVENTION

The present invention pertains generally to balloon catheters, and more particularly to a prefolded, ultracompact balloon catheter suitable for percutaneous insertion and placement in the aorta for counter-pulsation.

The use of intra-aortic catheters for counterpulsation treatment is by now a well-established part of cardiovascular care. As is well known to those in the art, an intra-aortic balloon catheter typically comprises a flexible catheter tube having mounted at one end a nondistensible but quite flexible balloon. Such catheters are inserted through an artery, usually the femoral artery, and advanced until the balloon is in the descending aorta, a relatively short distance downstream from the heart. The balloon is inflated and deflated repeatedly, out of phase with the heart, to reduce the load on the latter. This load reduction occurs in two ways: first, the deflation of the balloon as the heart pumps creates reduction of pressure within the aorta downstream from the heart, into which the heart displaces the pumped fluid (i.e., the impedance against which the heart works is reduced); and second, inflation of the balloon out of phase with the heart provides a supplemental pumping action to the circulation, especially to that of the coronary arteries.

Such balloons are commonly inserted in cases of extreme emergency. In order to reduce the trauma and preparation time involved in inserting such a balloon, a type of intra-aortic balloon catheter adapted to be inserted percutaneously (i.e., without surgery) was developed. The first such device is believed to be that disclosed in U.S. Patent 4,261,339, issued April 14, 1981 to Hanson and Wolvek, and commonly assigned herewith. In the device shown in that patent, the catheter tube terminates at the proximal end of the balloon chamber, and a thin support member, such as a wire, is placed in the balloon to support it. The support member is designed so as not to be telescoped into the catheter tube when the catheter is inserted into the body and advanced through the arterial system. The thinness of the wire or other support member permits the balloon to be compressed tightly against it to assume a configuration whose outer diameter is no greater than that of the catheter tube. As described in the above-mentioned patent, the wire is rotatable relative to the catheter tube, or alternatively, the distal tip of the balloon chamber is rotatable relative to the wire. Either arrangement permits the two ends of the balloon to rotate relative to each other and thus enables the balloon to be helically wrapped or twisted around the wire. Such rotation wraps the balloon down snugly against the support member. By use of the principles of the invention disclosed in that patent, percutaneously insertable intra-aortic balloon catheters on catheter tubes at least as small as 8.5 French have been made.

Intra-aortic balloon catheters of the type shown in the above-mentioned patent are believed to have been the first which could be inserted through a sufficiently small sheath to permit insertion into the femoral artery using the Seldinger catheterization technique. As a result, the size of catheter tube needed to assure fast enough gas flow to permit inflation and deflation over one hundred times per minute became the limiting factor in reducing the required sheath size.

Later work, however, culminated in a catheter in which the catheter tube was smaller in diameter than even the wrapped balloon, thus limiting further progress in the ever-proceeding effort to minimize the required sheath size. It would be desirable to provide a still smaller balloon catheter suitable for use for counterpulsation, in which the balloon is so compactly arranged around the support member that the limiting factor on further reductions in size is the required minimum diameter of the catheter tube rather than the balloon itself. The present invention permits a further reduction in the maximum diameter occupied by the balloon, and thus in the sheath size needed.

Rotatability like that described above is not necessary to make an intra-aortic balloon catheter percutaneously insertable. A system and method for percutaneous insertion of an intra-aortic balloon catheter in which relative rotation of the two ends of the balloon is not necessary are disclosed in U.S. Patent 4,327,709, issued May 4, 1982, to Hanson and Wolvek and commonly assigned herewith. In that patent it is disclosed that by making an intra-aortic balloon catheter in such a manner to allow the balloon to be collapsed to a state in which substantially all surfaces of the balloon extend longitudinally of the support member, as shown for example in Fig. 4 of that patent, to allow the balloon to be inserted into a sheath, percutaneous insertion can be achieved without rotation.

Other arrangements for making the balloon assume a compact configuration around the support member are known. For example, in one device, the balloon is asymmetric about the support member, extending mostly to one side of the latter, and the balloon membrane is furled around the support rod rather like a window shade around a roller. Furling in this manner, however, results in a series of oblique folds along the length of the furled balloon and, in addition, the asymmetric balloon is quite susceptible to the formation of creases or "puckers". Such puckers which form during wrapping result in relatively long lived creases which persist in the outer surface of the balloon after unwrapping, and may have a tendency to increase damage to blood components, especially platelets and red blood cells.

The most common shape for intra-aortic counterpulsation balloons is still a symmetric one, somewhat resembling that of a cigar, basically cylindrical with no or only slight taper along most of its length, and tapering more at the two ends. The tapering portions, like the asymmetrical balloon described above, pucker when wrapped, although such puckering is less pronounced in the standard symmetric shapes. It would be desirable to provide an intra-aortic balloon catheter in which the balloon can be made to assume a very compact configuration with the smoothest possible contour and as few creases or puckers as possible.

In the device described in U.S. Patent 4,261,339, wrapping is performed by gripping the distal tip of the balloon with one hand and the catheter tube with the other, and rotating those two portions of the catheter relative to each other around the longitudinal axis of the catheter. It is preferable, in such wrapping, to lightly grasp the balloon as the latter twists, moving the hand slowly toward the proximal end of the balloon just ahead of the twisting part of the balloon, to ensure a uniform wrap. If a wrapping knob, for example, provided in the proximal end of the catheter tube, is used to wrap the balloon around the support member, it is still considered advisable in some balloon catheters for the wrapping of the balloon to be guided with one hand, thus requiring a second person.

It would be desirable to provide a complete kit including such a catheter with the balloon prefolded about the support member and packaged in such a manner that the step of folding the balloon into its compact configuration is performed in the factory and thus need not be performed at the time of use.

While catheters having the asymmetric balloons described above have been prewrapped in holder devices which the user must remove before inserting the catheter into the sheath, the removal of the holder nonetheless requires a step which it would be desirable to eliminate to save time and reduce the possibility that the catheter may be damaged or contaminated during the maneuver.

## SUMMARY OF THE INVENTION

In accordance with one aspect the present invention provides a balloon catheter comprising:
a hollow catheter tube portion having a proximal end and a distal end;
a balloon membrane defining a balloon chamber, said balloon chamber having a distal portion and a proximal portion, said proximal portion of said balloon chamber being connected to said catheter tube portion in such a manner that the interior of said catheter tube portion communicates with the interior of said balloon chamber; and
an elongated member smaller in diameter than said catheter tube portion and disposed in said balloon chamber, said elongated member having a proximal end extending into said catheter tube portion and having a distal end attached to said distal portion of said balloon chamber, characterised by said balloon membrane being in a compact configuration capable of being formed by folding said balloon membrane along a longitudinal folding line, and wrapping said folded balloon membrane around said elongated member.

According to another aspect the invention provides a method of wrapping a balloon catheter of the type having a hollow catheter tube with a proximal end and a distal end, a balloon membrane defining a balloon chamber which has a proximal portion connected to the catheter tube in such a manner that the interior of the catheter tube communicates with the interior of the balloon chamber, an elongated member of smaller diameter than the catheter tube and disposed in the balloon chamber and having a proximal end extending into the catheter tube and a distal end attached to the distal portion of the balloon chamber, said method comprising wrapping the balloon membrane about the elongated member, characterised by the steps of: folding the balloon membrane along at least one folding line; and wrapping the folded balloon membrane about the elongated member.

Embodiments of the invention enable the following to be achieved:
a balloon catheter, such as an intra-aortic balloon catheter, in which the balloon is arranged around the support member so as to have a diameter less than the minimum diameter to which it has conventionally been possible to wrap such balloons;
a prefolded intra-aortic balloon catheter, eliminating the step of wrapping or folding the balloon from the procedure carried out by the physician when the catheter is used;
a prefolded balloon catheter which does not require the removal of the folding means or retainer from the balloon when the balloon catheter is used;
a prefolded balloon catheter, and a method for folding a catheter, that result in a smaller and smoother contour and end-on profile of the balloon, to reduce any tendency of the balloon to damage blood components;

a prefolded intra-aortic balloon catheter in which the balloon does not retain major creases for any significant time after being unfolded;

a catheter having the balloon arranged more tightly and to a smaller diameter about the support member than has conventionally been possible, in such a manner that, even without a vacuum being pulled on the balloon, the balloon will retain its compact configuration sufficiently to permit easy percutaneous insertion without any folding, wrapping or similar manipulation by the user;

a method of folding an intra-aortic balloon in such a manner as to minimize the number of oblique folds on the surface of the folded balloon;

a method of folding an intra-aortic balloon in such a manner as to minimize puckering of the balloon; and

an intra-aortic balloon capable of being inserted percutaneously with less resistance than has hitherto been possible, to reduce still further the possibility of trauma to the artery as a result of the insertion.

Conveniently the balloon, which is preferably symmetric about the support member, instead of being twisted or wrapped spirally about an elongated member which extends through the balloon chamber is folded as follows. First, the balloon is folded along one or more longitudinal fold lines to define longitudinal creases in the balloon membrane ("longitudinal" is used throughout the summary, detailed description of the preferred embodiments, and claims to mean "substantially parallel to the proximal-distal direction", "fold line" is used herein to refer to a line along which a folding operation is performed, and "crease" is used hereinafter to mean a bend produced in the balloon membrane by folding). After this folding, the balloon membrane is wrapped around a longitudinal line, preferably around the elongated member, which may be, for example, an inner lumen. Preferably, one or more retaining devices are then placed over the balloon to hold it in the desired configuration.

Most preferably, the balloon is flattened before the folding, and the flattened balloon membrane is folded over on to itself along a longitudinal line to form a flat four-fold thickness of the membrane. Then, the four-fold thickness is rolled up or wrapped around the elongated member. This version of the invention results in the balloon having a particularly compact configuration and a very smooth end-on profile.

The longitudinal folding has several advantages. First, it eliminates any need for an asymmetric balloon (although an inner lumen or other support member and the longitudinal fold line do not by any means need to coincide with or even to be near the axis of symmetry, if any, of the balloon chamber). Second, the present arrangement is calculated to reduce possible damage to blood components that might result from the balloon receiving long-lived creases, since the longitudinal creases defined in the balloon membrane by the folding have been found to disappear very quickly, within a matter of minutes, after pumping has begun.

Moreover, the smoother contour presented by the present invention at the distal end of the device facilitates insertion through a sheath of a given size. The actual diameter of the balloon when wrapped in this manner is reduced as much as 20-30 percent beyond what is possible using the conventional spiral wrap, further facilitating insertion and reducing the resistance encountered by the physician.

The foregoing features and advantages of the invention will be more fully understood and appreciated from a consideration of the following detailed description of several preferred embodiments thereof, taken in conjunction with the accompanying drawings.

## BRIEF DESCRIPTION OF THE FIGURES

In the following figures, like elements are denoted by like reference characters.

Fig. 1 is a diagrammatic view of a preferred embodiment of an intra-aortic balloon of a type suitable for percutaneous insertion.

Fig. 1A is a cross-sectional view of a detail of Fig. 1.

Fig. 2 is a view of the distal portion of a conventional percutaneously-insertable intra-aortic balloon catheter, with the balloon twisted in a known manner about the support member.

Fig. 2A is a view taken from line 2A-2A in Fig. 2.

Fig. 3 is a detail of the distal portion of the intra-aortic balloon catheter of Fig. 1, illustrating the first step in wrapping the balloon catheter according to the most preferred form of the invention.

Fig. 3A is a view taken from line 3A-3A in Fig. 3.

Fig. 4 is a view like that of Fig. 3, showing the following steps of wrapping the balloon.

Fig. 4A is a view taken from line 4A-4A in Fig. 5.

Fig. 5 is a similar view of the catheter of Fig. 3, with the balloon fully wrapped, and showing a retainer used in packaging the prewrapped catheter, according to one aspect of the invention.

Fig. 5A is a view taken from line 5A-5A in Fig. 5.

Fig. 6 is a view similar to Fig. 3, with a pair of retainers in place over the wrapped balloon.

Fig. 7 is a view showing a portion of a kit including a prewrapped intra-aortic balloon catheter of the invention.

Figs. 8 and 8A are views like that of Fig. 7, with the catheter removed from the package.

Figs. 9 and 10 are cross-sectional views of the balloon folded in other manners in accordance with the invention.

## DETAILED DESCRIPTION OF THE PREFERRED EMBODIMENTS

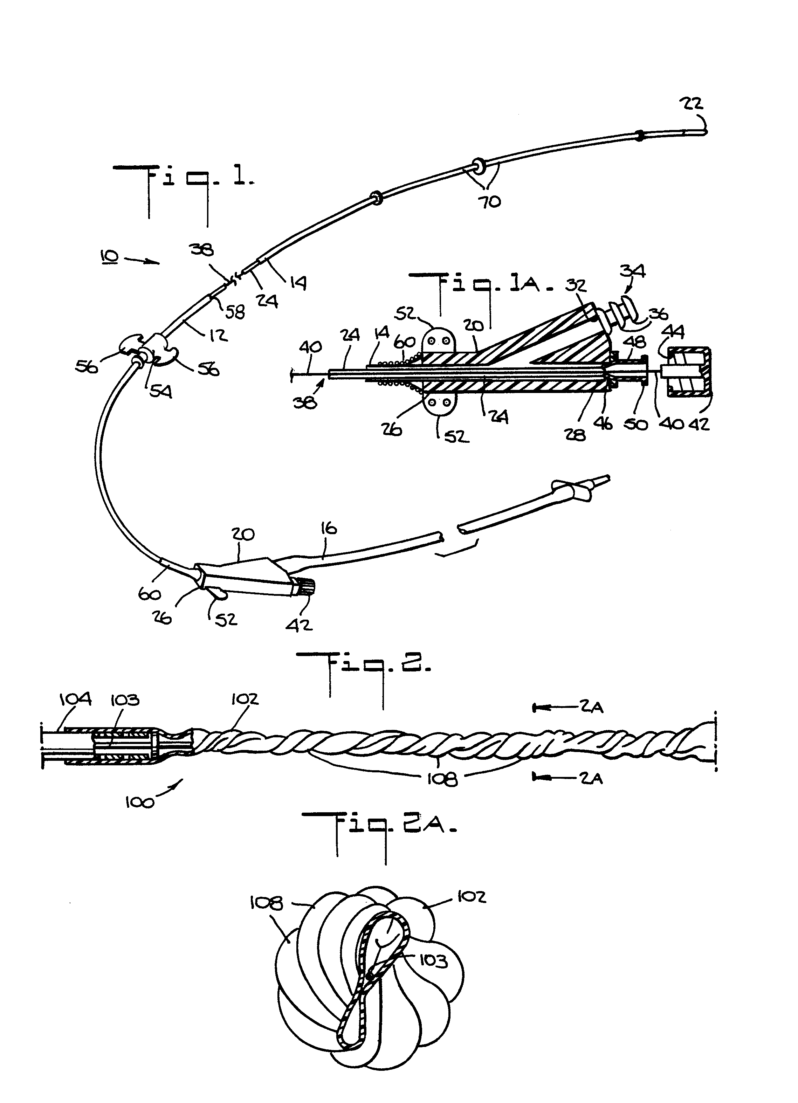

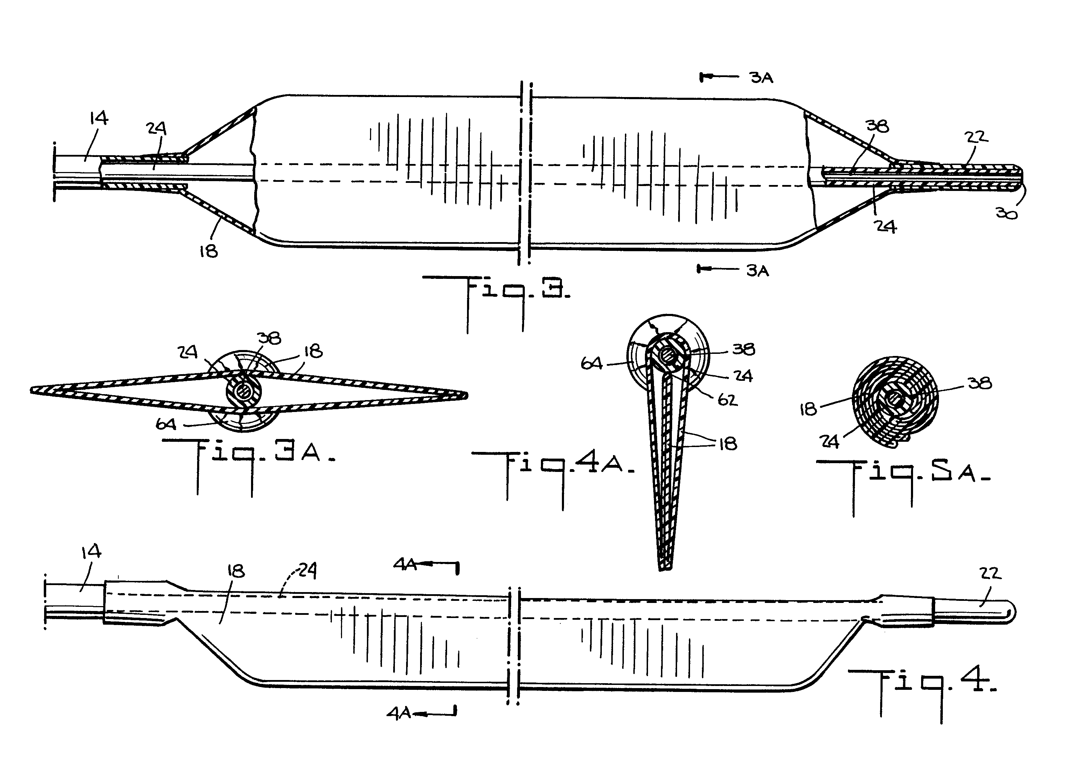

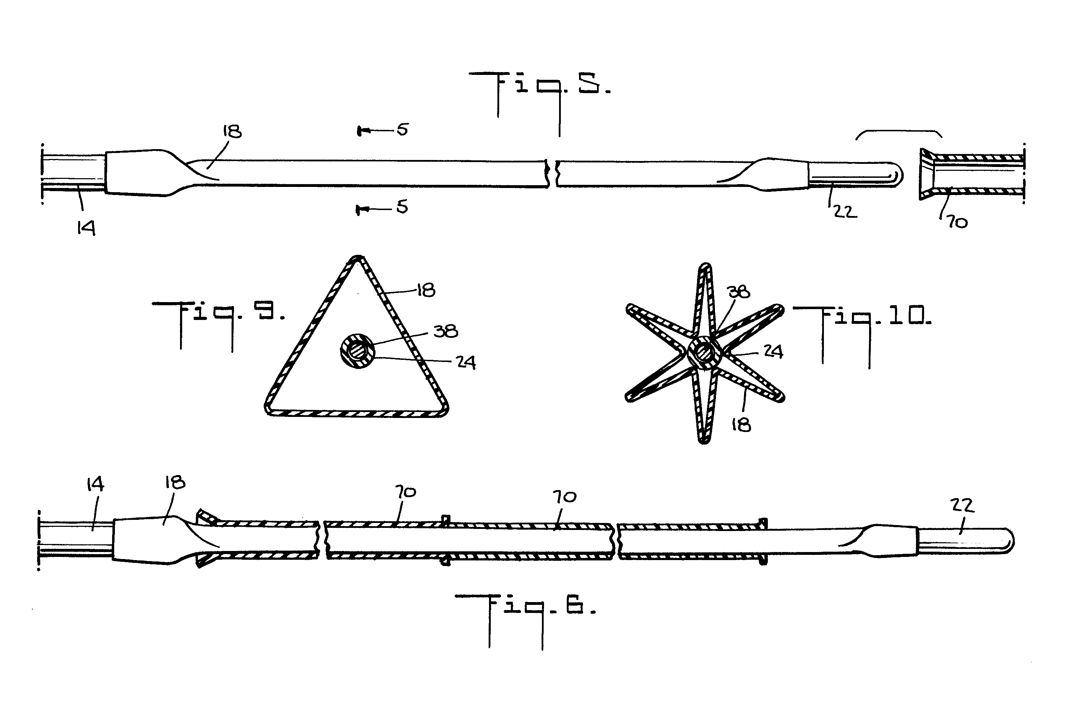

Figure 1 shows one preferred embodiment of a balloon catheter 10 according to the invention. The catheter 10 has a catheter tube portion 12 which includes a hollow tube 14 made of a flexible material, such as polyurethane, and has its proximal end connected via a second hollow tube 16 to a source (not shown) of an inflating fluid such as carbon dioxide or helium, and its distal end connected to a balloon chamber 18 formed by a membrane of a flexible material. Preferably, that material has no substantial elasticity and may, for example, be polyurethane. The catheter tube portion 12 also includes a connector 20, which may be made of plastic, to which the proximal end of the tube 14 is attached. The distal end of the balloon 18 (see Fig. 3) is attached to a preferably radiopaque tip assembly 22. An additional tube 24, also preferably of plastic to make it flexible, is provided inside the outer catheter tube 14 and balloon chamber 18, and has its distal tip secured to the tip assembly 22 and its proximal end passing through a first opening 26 of the connector 20 (see Fig. 1A) and being secured to a second opening 28 thereof. The tip assembly 22 (see Fig. 3) has an axial bore 30 via which the interior of the inner tube 24, or inner lumen, communicates with the exterior of the instrument. Thus the inner lumen 24 defines a passage from the connector 20 to the tip assembly 22, fluid-tightly sealed from the interior of the connector 20, the outer tube 14 and the balloon chamber 18. The inner lumen 24 is used by the physician in known fashion to inject dyes or drugs, insert the catheter using a guidewire, monitor the aortic pressure, etc.

A third opening 32 of the connector 20 is provided at its exterior with a barbed projection or nipple 34, over which the second tube 16 is fitted. The barbs 36 hold the tube 16 in place. This opening places the second tube 16 in communication with the outer lumen defined between the inner tube 24 and the outer tube 14. Via the outer lumen, the pumping gas is moved into and out of the balloon chamber 18.

A means may be provided to impart stiffness against axial compression, during insertion, to the balloon chamber 18. In the embodiment shown, a stylet 38, which in the embodiment shown illustratively comprises a stainless steel wire 40 having a blunted distal end, and a plastic knob 42 to which the proximal end of the wire is secured, is provided in the inner lumen 24. The end of the wire 40 is, for example, glued in a bore in the knob 42. The stylet 38, when in place, extends into the bore of the tip assembly 22 and provides stiffness to aid in inserting the catheter 10 and advancing it through the arterial system.

The stylet 38 is removed from the catheter after the latter is in place, if the physician wishes to use the inner lumen, or if insertion is to be carried out using a guidewire instead of the stylet, it is removed before insertion. To hold the stylet 38 in place, the knob 42 has a second bore 44 formed in its distal face, and the lateral surface of bore 44 is threaded. The connector 20 has, at its second opening 28, a small hollow block 46 with a short cylindrical flange 48 at the proximal end of which are two diametrically opposing tabs 50. The threads of the knob engage the tabs to hold the stylet 38 in place.

The connector 20 also has two oppositely-extending tabs 52 provided with two small holes each for suturing the connector 21 to the skin. A plastic sleeve 54, slidable along the outer tube 14, forms a seal in a known manner with the sheath upon insertion of the balloon catheter into a patient, and is also provided with two additional apertured tabs 56 for the same purpose.

Marks 58 are provided on the inner tube 24 at intervals to aid the physician in determining when the instrument 10 has been advanced to the correct position in the aorta, the outer tube 14 being transparent or at least translucent for this purpose.

A coil spring 60 at the distal side of the connector 20 relieves bending strain at the junction of the connector 20 with the outer tube 14 without impairing flexibility.

While the balloon 18 may have any of several different shapes, it is preferred for the balloon of an intra-aortic balloon catheter to be relatively long and slender, roughly cylindrical for the greater part of its length and significantly tapered only toward the ends. In the version illustratively shown in Fig.

1, for example, the balloon 18 is tapered roughly conically from the long middle portion to the tip assembly at the distal end and to the outer surface of the catheter tube at the proximal end.

An intra-aortic balloon catheter 100 (shown only partially; see Fig. 2) of the type disclosed in U.S. Patent 4,261,339 is, as stated above, prepared for insertion by wrapping or twisting the balloon membrane 102 around a central support rod or other member. Since the catheter 100 is constructed so as to make the two ends of the balloon chamber 102 free to rotate relative to each other, it is possible by such rotation to wrap the balloon membrane 102 closely about the support member, twisting the balloon membrane spirally, as shown in Figs. 2 and 2A. This greatly reduces the maximum cross-sectional area occupied by the balloon chamber, and permits the catheter to be inserted through a smaller sheath than would be required to accommodate the balloon if the catheter tube extended through the balloon chamber.

In the device described in U.S. Patent 4,261,339, wrapping is performed by gripping the distal tip of the balloon 102 with one hand and the catheter tube 104 with the other, and rotating those two portions of the catheter 100 relative to each other around the longitudinal axis of the catheter. It is preferable, in such wrapping, to lightly grasp the balloon as the latter twists, moving the hand slowly toward the proximal end of the balloon just ahead of the twisting part of the balloon, to ensure a uniform wrap. If a wrapping knob (not shown), for example, at the proximal end of the catheter tube, is used to wrap the balloon around the support member, it is still often considered advisable for the wrapping of the balloon to be guided with one hand, thus requiring a second person.

The present invention makes it possible to eliminate the need for such two-handed wrapping procedures on the part of physicians and their assistants.

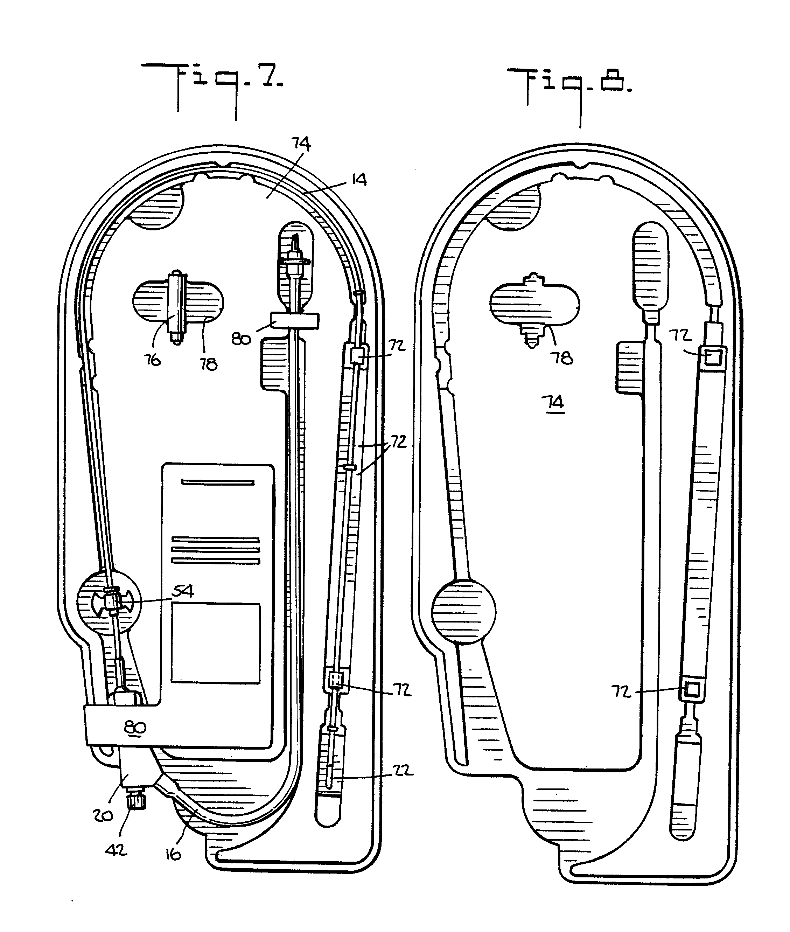

As shown in Figs. 3 and 3A, the balloon 18 is initially preferably pressed as flat as possible, for example by pulling a vacuum on it, although especially near the tip assembly 22 and the junction with the catheter tube 14 (especially the latter since it is of greater diameter then the tip assembly), the balloon membrane 18 will not be entirely flat. The flattened balloon membrane is then folded over onto itself along a longitudinal line to form a four-fold thickness of the membrane and a longitudinal crease 62 in the membrane, as shown in Figs. 4 and 4A. In the example illustrated, the longitudinal fold-line 62 lies along the inner tube 24, although that is by no means necessary according to the broad invention. Again, the regions of the balloon membrane nearest the ends of the balloon chamber 18, because of their connection to the typically cylindrical catheter tube and tip assembly, are not entirely flat, but have some folds or puckers 64 formed in them. These, however, are relatively small, since the longitudinal folding has divided the balloon membrane into four regions each of which contains only a small amount of excess material at each end of the balloon 18 available to form puckers 64. After the folding, the quadruple thickness of balloon membrane is wrapped up, or furled, around the support member (or more generally, around a second longitudinal line, although the most compact configuration by far is obtained when the inner tube 24 is the center of this furling).

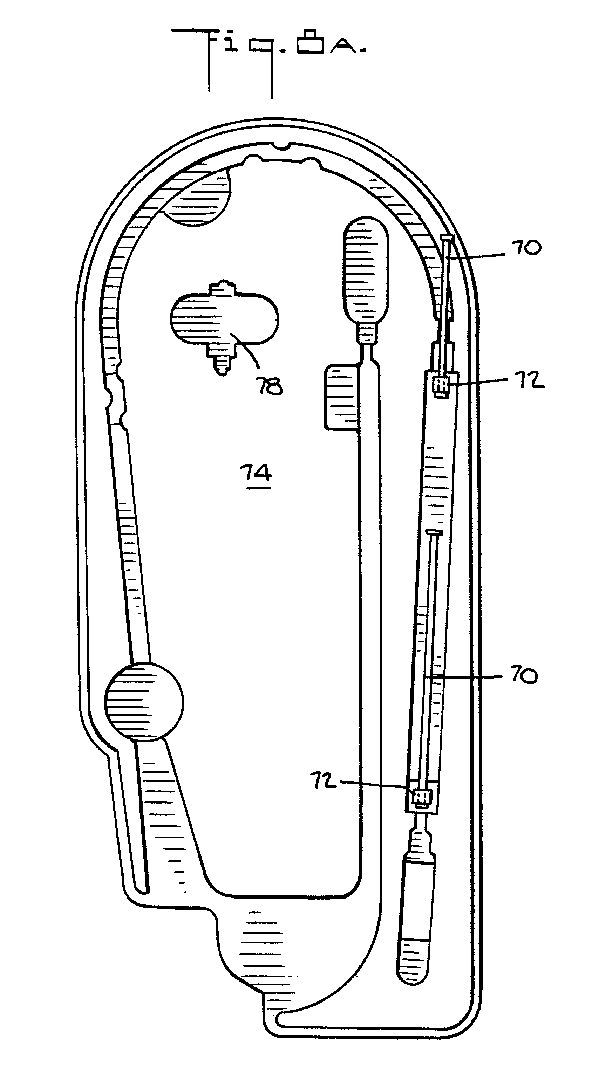

The resulting configuration, as seen from Figs. 5 and 5A, is very compact, and very smooth, and especially presents a very smooth end-on profile as seen from the distal end of the instrument. With, e.g., a 40cc. balloon chamber, this configuration is smaller than the outer diameter of even an 8.5 French catheter tube. The compactness and the smooth front profile greatly reduce the resistance felt by a physician in inserting the instrument through a sheath of a given size. This means that the physician need not rotate or otherwise manipulate the catheter when inserting it into the sheath. Alternatively, the extra compactness obtained in this fashion allows the use of as small a sheath as the catheter tube will permit.

In addition, the smooth front profile of the prefolded balloon catheter, especially compared to the relatively large spiral ridges 108 which occur in a conventionally wrapped balloon catheter - (compare Figs. 2A and 5A), is believed to reduce the tendency of the catheter to damage the internal walls of the blood vessels or delicate blood components such as platelets and red blood cells.

The smaller diameter and lower insertion resistance also make it easier to advance the balloon catheter through a tortuous arterial system or one having a large amount of plaque, whether the balloon catheter is a dual lumen device like that illustrated, or a single lumen instrument with a wire or other support member in place of the inner tube. Lower resistance to insertion also permits the physician to use less force to insert and advance the catheter, which also reduces the risk of trauma to the artery walls.

After the balloon has been folded in the manner described to obtain the configuration shown in Fig. 5, one or more retainer devices, such as the sleeve-like retainers 70 illustrated, are slipped over the distal tip assembly 22 and the balloon membrane 18 to hold the latter in that configuration. Preferably, retainers 70 (using more than one facilitates their application to and removal from the balloon) are attached, as by plastic clips 72 glued or otherwise affixed in place, to a packaging tray 74 designed to receive the balloon catheter 10 in a predetermined configuration for sterilization and shipping (see Figs. 7 and 8). This allows the balloon catheter to be removed from the retainers and the tray simultaneously (see Fig. 8A). Also, the tray is sealed with a preferably transparent cover to maintain sterility until the package is opened just prior to use. Accessories such as a one-way valve 76 which may be used, as is well known, in pulling a vacuum on the balloon preparatory to insertion may also be packaged in recesses 78 provided for them in the embodiment of the tray illustrated. Paper strips 80 adhered to the tray, or any other suitable expedients, may further be provided to retain the catheter in place in the tray.

The retainers may, if desired, be removable from clips 72 so that the catheter can be removed from the tray with the retainers still in place over the balloon (see Fig. 8; compare with Fig. 8A). Alternatively, the balloon catheter can be prefolded according to the present invention and shipped without retainer sleeves or the like.

The physician thus receives the balloon catheter ready to be removed from the tray and inserted, without any wrapping or similar manipulation required of the user. Once the retainers, which are smaller in diameter than the sheath to be used with the cathether have been in place for a short period of time, the balloon remains tightly enough folded after their removal to be inserted into a sheath and it is believed that this is true, even if no vacuum is pulled on the catheter. Thus, according to this aspect of the invention, the user receives a catheter that is ready to insert, with minimal preparation, once it is removed from its package and immersed in saline solution.

It has also been found that, although the balloon easily retains its folded configuration even without the retainers remaining in place and without a vacuum, any creases in the balloon membrane due to the folding disappear completely within a relatively short period, such as a few minutes, and are not believed to affect either counterpulsation or any risk of clot-formation.

While the invention has been described with reference to an embodiment in which the balloon membrane is folded about an axial fold-line that lies along the inner tube, it is also within the scope of the invention to fold the balloon membrane along two or more lines, as shown in Figs. 9 (three lines) and 10 (a star-shaped configuration).

As described hereinabove the two retainers 70 are retained on the tray 74 by the clips 72 when the balloon catheter is removed from the tray, as depicted in Fig. 8A. In a preferred arrangement the upper retainer at least is capable of limited sliding movement relative to the clips and when the balloon catheter is removed, by upward movement relative to the clips as seen in Figs. 7 and 8A, displacement of the lower retainer with the balloon chamber is arrested by the lower clip 72 before such displacement of the upper retainer is arrested by the upper clip, whereby the balloon chamber begins to slide relative to the lower retainer before it begins to slide relative to the upper retainer.

Although the present invention has been described with reference to several preferred embodiments thereof, many variations and modifications thereof will be readily apparent to those skilled in the art from the foregoing description. Accordingly, the scope of the invention is to be limited, not by the details of the preferred embodiments illustratively described herein, but only by the terms of the appended claims.

## Claims

1. A balloon catheter comprising:
a hollow catheter tube portion (12) having a proximal end and a distal end;
a balloon membrane (18) defining a balloon chamber, said balloon chamber having a distal portion and a proximal portion, said proximal portion of said balloon chamber being connected to said catheter tube portion in such a manner that the interior of said catheter tube portion communicates with the interior of said balloon chamber; and
an elongated member (24) smaller in diameter than said catheter tube portion and disposed in said balloon chamber, said elongated member having a proximal end extending into said catheter tube portion and having a distal end attached to said distal portion of said balloon chamber, characterised by said balloon membrane (18) being in a compact configuration capable of being formed by folding said balloon membrane along a longitudinal folding line, and wrapping said folded balloon membrane around said elongated member.

2. A balloon catheter as claimed in claim 1, wherein said configuration is one capable of being formed by folding said balloon membrane (18) along a single longitudinal folding line (62) to define a four-fold thickness of said balloon membrane, and wrapping said four-fold thickness of said balloon membrane around said elongated member - (24).

3. A balloon catheter as claimed in claim 1 or 2, further comprising a retainer (70) having an inner diameter smaller than the outer diameter of said catheter tube portion (12), said retainer being fitted over said wrapped balloon membrane so that the interior of said retainer tends to hold said balloon membrane in said configuration.

4. A balloon catheter as claimed in claim 1, 2 or 3, wherein said balloon chamber has a central axis about which it is substantially symmetric, wherein said elongated member (24) is disposed in said balloon chamber so as to lie substantially along said axis of said balloon chamber, and wherein said axis of said balloon chamber coincides with the longitudinal folding line along which said balloon membrane is folded.

5. A balloon catheter as claimed in any of claims 1 to 4, wherein said elongated member (24) is hollow.

6. A balloon catheter as claimed in any of claims 1 to 4, wherein said elongated member is solid.

7. A balloon catheter as claimed in any of claims 1 to 6, wherein the longitudinal folding line lies along said elongated member.

8. A balloon catheter as claimed in claim 5, further comprising a stylet (38) in said hollow elongated member (24).

9. A kit comprising:

A) a balloon catheter having:

1) a catheter tube (14) having a proximal and a distal end;

2) a balloon membrane (18) defining a balloon chamber, said balloon chamber having a distal portion and a proximal portion, said proximal portion of said balloon chamber being connected to said catheter tube (14) in such a manner that the interior of said catheter tube communicates with the interior of said balloon chamber; and

3) an elongated member (24) smaller in diameter than said catheter tube portion and disposed in said balloon chamber, said elongated member having a proximal end extending into said catheter tube and having a distal end attached to said distal portion of said balloon chamber; and

B) packaging for the balloon catheter; characterised by:

said balloon membrane (18) being in a compact prewrapped configuration capable of being formed by folding said balloon membrane along at least one longitudinal folding line, and then wrapping said folded balloon membrane around said elongated member (24); and

said packaging comprising a packaging device (74) including means (70, 72) for retaining said folded and wrapped balloon catheter in place in said packaging device.

10. A kit as claimed in claim 9, wherein said retaining means comprises at least one retainer (70) having an interior so shaped and dimensioned as to fit over at least a portion of said balloon chamber to retain said balloon membrane (18) in said configuration.

11. A kit as claimed in claim 10, wherein said packaging device further comprises a tray (74) formed to receive said balloon catheter (10) thereon at a predetermined position, said balloon catheter being received on said tray at said position.

12. A kit as claimed in claim 11, wherein said retainer (70) is attached to said tray (74) in such a manner as to permit said balloon catheter to be removed from said tray and said retainer without separating said retainer from said tray.

13. A kit as claimed in claim 11, wherein said retainer (70) is attached to said tray (74) in such a manner as to be removable from said tray with said balloon catheter.

14. A kit as claimed in any one of claims 9 to 13, wherein said elongated member (24) of said balloon catheter is solid.

15. A kit as claimed in any of claims 9 to 13, wherein said elongated member of said balloon catheter is hollow.

16. A kit as claimed in claim 15, further comprising a stylet (38) received in said hollow elongated member (24).

17. A kit as claimed in any one of claims 9 to 16, wherein the longitudinal folding line lies along said elongated member.

18. A kit as claimed in claim 11, wherein said retaining means further comprises a first retainer (70) and a second retainer (70), said second retainer being distal to said first retainer, and wherein said first retainer is so shaped and dimensioned as to fit over a first portion of said balloon chamber to retain said first portion in said pre-wrapped configuration, while said second retainer is so shaped and dimensioned as to fit over a second portion of said balloon chamber to retain said second portion in said pre-wrapped configuration.

19. A kit as claimed in claim 18, wherein said retainers (70) are attached to said tray (74) in such a manner as to permit removal of said balloon catheter (10) from said tray by longitudinal movement of said balloon catheter out of said retainers such that said balloon catheter is released by said first retainer before it is released by said second retainer.

20. A kit as claimed in claim 18, wherein said retaining means further comprises a first clip means (72) associated with said first retainer in such a manner as to permit balloon catheter removal from said tray without removal of said first retainer from said tray.

21. A kit as claimed in claim 18, wherein said retaining means further comprises a second clip means (72) associated with said second retainer (70) in such a manner as to permit balloon catheter removal from said tray without removal of said second retainer from said tray.

22. A kit as claimed in claim 20, further comprising a second clip means (72) associated with said second retainer (70) in such a manner as to permit balloon catheter removal from said tray without removal of said second retainer from said tray.

23. A kit as claimed in claim 22, wherein said first and second retainers are hollow tubes (70) and the prewrapped balloon chamber is retained within said hollow tubular retainers wherein said balloon chamber is removed from said tray by sliding said balloon chamber from within said tubular retainers, wherein said first and second clip means are positioned so that during removal of said balloon chamber from said tray said balloon chamber begins to slide relative to said second retainer before it begins to slide relative to said first retainer.

24. A method of wrapping a balloon catheter of the type having a hollow catheter tube (14) with a proximal end and a distal end, a balloon membrane (18) defining a balloon chamber which has a proximal portion connected to the catheter tube in such a manner that the interior of the catheter communicates with the interior of the balloon chamber, an elongated member (24) of smaller diameter than the catheter tube and disposed in the balloon chamber and having a proximal end extending into the catheter tube and a distal end attached to the distal portion of the balloon chamber, said method comprising wrapping the balloon membrane about the elongated member, characterised by the steps of:
folding the balloon membrane (18) along at least one folding line (62); and
wrapping the folded balloon membrane about the elongated member (24).

25. A method as claimed in claim 24, wherein the balloon membrane is folded along a single folding line (62) which lies substantially along the elongated member (24).

26. A method as claimed in claim 24 or 25, further comprising the step of flattening the balloon membrane (18) before said folding step.

27. A method as claimed in claim 26, wherein said flattening step is performed by applying suction to the interior of the balloon chamber.

28. A method for compactly wrapping a balloon catheter of a type having a hollow catheter tube - (14) having a proximal end and a distal end, a balloon membrane (18) defining a balloon chamber which has a proximal portion connected by means of a first connection to the catheter tube in such a manner that the interior of the catheter tube communicates with the interior of the balloon chamber, an elongated member (24) disposed in the balloon chamber and having a proximal end extending into the catheter tube and a distal end attached by means of a second connection to the distal portion of the balloon chamber, said method comprising wrapping the balloon membrane about the elongated member, characterised by the steps of:
folding the balloon membrane (18) along at least one folding line (62) to define therein a plurality of temporary creases to divide the balloon membrane into a plurality of longitudinal regions which each extend from the first connection, at the proximal portion of the balloon chamber, to the second connection, at the distal portion of the balloon chamber, and each of which regions includes, at each end of the balloon chamber, only a portion of the circumference of the balloon membrane; and
wrapping the folded balloon membrane about the elongated member (24) to cause the balloon membrane to assume a compact configuration about the elongated member.

29. A method as claimed in claim 28, wherein said folding step consists of folding the balloon membrane (18) along a single longitudinal folding line (62).

30. A method as claimed in claim 27 or 28, further comprising the step of flattening the balloon membrane (18) before said folding step.

31. A method as claimed in claim 28, 29 or 30, wherein said folding step is performed in such a manner that at least some of the temporary creases lie along the elongated member.

Fig. 1.

Fig. 1A.

Fig. 2.

Fig. 2A.

Fig.3.

Fig.3A.

Fig.4.

Fig.4A.

Fig.5A.

Fig. 5.

Fig. 6.

Fig. 9.

Fig. 10.

Fig.7.

Fig.8.

**Fig. 8A.**

| Category | Citation of document with indication, where appropriate, of relevant passages | Relevant to claim | CLASSIFICATION OF THE APPLICATION (Int. Cl.4) |
|---|---|---|---|
| | **DOCUMENTS CONSIDERED TO BE RELEVANT** | | EP 86308643.5 |
| A | EP - A1 - 0 047 465 (KONTRON CARD.) <br> * Totality; especially claims 1,3,4 * | 1,9, 24,28 | A 61 M 25/00 <br> A 61 M 1/10 |
| A | US - A - 4 444 186 (S.WOLVEK et al.) <br> * Totality * | 1,9, 24,28 | |
| D,A | US - A - 4 261 339 (B.L.HANSON et al.) <br> * Totality * | 1,9, 24,28 | |
| D,A | US - A - 4 327 709 (B.L.HANSON et al.) <br> * Totality * | 1,9, 24,28 | |
| A | US - A - 4 402 307 (B.L.HANSON et al.) <br> * Totality * | 1,9, 24,28 | TECHNICAL FIELDS SEARCHED (Int. Cl.4) <br><br> A 61 M 1/00 <br> A 61 M 25/00 |
| P,A | US - A - 4 576 142 (P.SCHIFF) <br> * Totality; especially fig. 2,2a-2d,3,3a,4a * | 1,9, 24,28 | A 61 M 29/00 |

The present search report has been drawn up for all claims

| Place of search | Date of completion of the search | Examiner |
|---|---|---|
| VIENNA | 09-04-1987 | LUDWIG |